# EUROPEAN PATENT APPLICATION

(11) **EP 0 718 399 A1**
(43) Date of publication of application: **26.06.1996**
(21) Application number: 94402983.4
(22) Date of filing: 21.12.1994
(51) Int. Cl.: C12N 1/02, C12N 1/38

(54) **Permentation broth composition**

(71) Applicant: DAICEL CHEMICAL INDUSTRIES, LTD., Sakai-shi, Osaka (JP)
(72) Inventor: Mathias, Rolland, F-75005 Paris (FR); Akira, Fujikawa, Yokohama-shi, Kanagawa (JP)
(74) Representative: Le Guen, Gérard

(57) **Abstract**

A fermented broth composition containing a biodegradable filamentous organic substance having an average fiber diameter of from 0.7 to 10 µm and a filamentous actinomyces. By separating and purifying this fermented broth, the target substance is obtained. Filtration can be effected at an extremely high efficiency without using any filter-aid. Further, the filtration residue is composed mainly of harmless organic materials and thus reusable as fertilizers, feeds, etc. When the filtration residue is incinerated, furthermore, only a small amount of ash is formed, which reduces the load on the incinerator.

## Description

This invention relates to the field of fermentation technology. It provides a technique which is highly useful in the step of separating the target product and useless matters from a fermented broth.

Fermentation is an important process in the field of industrial biotechnology. A number of valuable products such as vitamins, amino acids, steroid hormones, alkaloids and antibiotics are produced via fermentation.

There have been employed various microorganisms including fungi and bacteria such as actinomyces in fermentation processes. Fungi and actinomyces usually exist in the form of a filamentous fungus. Examples of the fungi which have been most frequently employed in the fermentation processes include those belonging to the genera Penicillium, Cephalosporium, Aspergillus, Rhizopus and Mucor. As actinomyces, those belonging to the family Streptomyces have been frequently used. For example, there have been used Streptomyces erythraeus, Streptomyces aureofaciens and Streptomyces griseus for producing various antibiotics and enzymes.

It is necessary in a fermentation process to treat the fermented broth containing the target product after the fermentation step so as to isolate the target product in an appropriate form, usually as a pure crystalline product. However the fermented broth contains various useless solid matters including unmetabolized or incompletely metabolized feedstocks which are insoluble in water (mainly comprising vegetable meals and calcium carbonate), antifoams employed in the fermentation step (oils, silicone) and other residues. The fermented broth also contains a microorganism added for producing the target substance.

In the process for obtaining the target substance from the fermented broth, it is first required to completely remove these solid matters including the microorganisms contained in the fermented broth.

In the prior art, a microorganism which is not necessary any longer after the completion of the fermentation is removed from the fermented broth by filtering off together with other useless solid residues as described above. However this separation and purification technique employed in the prior art suffers from a number of problems.

A first problem resides in the treatment of the useless matters. The matters to be removed from the fermented broth by filtration are fine and slimy ones. When pressure is applied to the fermented broth in the filtration step, therefore, a compressible cake with a poor liquid permeability is formed on the filter face, which causes an extreme decrease in the filtration rate. It is therefore necessary to use a filter-aid in order to form a highly permeable cake. Typical examples of the filter-aids to be used in the filtration of a fermented broth are inorganic materials such as Diatomite and Perlite. These filter-aids are used in precoating or body feeding. However there arises another problem in the post-treatment of a cake formed by using such a filter-aid comprising an inorganic material. Specifically, such a cake cannot be used as a fertilizer or an animal feed and no biological waste treatment system is applicable thereto. It is also troublesome to incinerate such a cake, since a large amount of ash is formed or an incineration device is damaged thereby. Accordingly the cost for treating these cakes thus separated has been increasing year by year.

A second problem resides in that it is unavoidably needed in general to use a filter-aid for precoating, which relates to the first problem as described above. The addition of the step of precoating causes a rise in the cost for the separation and purification. Further, the filter-aid should be used in an elevated amount, which increases the load in the waste treatment.

A third problem resides in changes in the optimum conditions for the separation and purification due to the decomposition and morphological changes in the cells with the passage of time in the course of the separation and purification. Although the decomposition and morphological changes in the cells with the passage of time are affected by the types and amounts of the feedstocks and antifoams, they are more largely affected by the temperature and pH value of the fermented broth and the composition of the employed solvents. Accordingly, the separation and purification should be carried out by highly skilled and experienced workers and it is very difficult to obtain the target product of excellent qualities in a steady yield.

The present inventors have conducted extensive studies in order to solve the above-mentioned problems. As a result, they have successfully completed the present invention.

The invention provides a fermentation broth composition comprising a biodegradable, filamentous organic substance having an average fiber diameter of from 0.7 to 10 µm and a filamentous actinomyces as a microorganism to employ for the fermentation and then a process for conducting a fermentation reaction with a filamentous actinomyces as the microorganism, characterized by adding a biodegradable filamentous organic substance having an average fiber diameter of from 0.7 to 10 µm to the microorganism, then conducting the fermentation and isolating desired components from the product mixture by way of filtration.

It is preferable that the actinomyces is Streptomyces erythraeus or Streptomyces aureofaciens; a weight ratio of the actinomyces to the biodegradable filamentous organic substance ranges from 0.01 to 1.0; the biodegradable filamentous organic substance is a cellulose fiber or a chitin fiber; a weight distribution of filaments having an average fiber diameter of from 1.7 to 7.0 µm in said biodegradable filamentous organic substance is at least 50%.

It is preferable that the biodegradable filamentous organic substance may be added at any step before the fermentation.

Moreover the present invention provides a fermented broth composition containing a biodegradable filamentous organic substance having an average fiber diameter of from 0.7 to 10 µm and a filamentous actinomyces as a microorganism employed for the fermentation and a process for treating a fermented broth composition characterized in that, in any step of a fermentation process with the use of a microorganism, a biodegradable filamentous organic substance having an average fiber diameter of from 0.7 to 10 µm is added to the fermentation medium followed by the separation and purification of the fermented broth obtained by the fermentation.

The fermented broth composition of the present invention is one which can be separated and purified without using any filter-aid such as Diatomite or Perlite. Further, the waste remaining after separating useful components therefrom is biodegradable, which makes a large cost for the waste treatment unnecessary. In the case of the conventional fermented broth compositions, the solid matters to be removed therefrom are fine ones which cannot be caught by usual techniques for separation and purification. Thus it is necessary to use a filter-aid such as Diatomite or Perlite. When the solid matters to be filtered off are slimy ones, a membranous filter layer having a poor permeability is formed in the step of the filtration. In this case, it is also necessary to use, for example, Diatomite or Perlite. In addition, it is substantially impossible to achieve the optimum conditions for the separation and purification because of the change in the system caused by chemicals and solvents which are used to cease the fermentation prior to the separation and purification (i.e., changes in pH value, solubilities of solid matters, etc.) and modifications in the system due to the autodecomposition which proceeds depending on time and temperature (i.e., conversion of the solid matters into smaller and slimier ones, etc.).

In the fermented broth composition according to the present invention, in contrast thereto, solid matters originating in a microorganism, the separation and purification of which are particularly troublesome among the solid matters to be removed, have a non-slimy and filamentous structure of high chemical and physical stabilities. Therefore it requires no filter-aid such as Diatomite or Perlite and the optimum conditions for separating and purifying the same can be easily achieved. Thus the waste thereof, which is free from any inorganic material such as Diatomite or Perlite, can be easily treated. Further, it enables the fermentation plant to be operated under the optimum conditions whereby a product of excellent qualities can be produced in a high yield.

Now the present invention will be described in greater detail.

The present inventors prepared fermented broth compositions containing filamentous organic substances of various fiber diameters and repeated separation and purification tests. As a result, they have surprisingly found out that the separation and purification efficiencies can be remarkably improved, even though the precoating step is completely omitted, only when a filamentous substance having an average fiber diameter within a specific range is used. The present invention has been completed based on this finding.

It has been clarified that the reason for the remarkable improvement in the separation and purification efficiencies in the fermented broth composition of the present invention is as follows. Namely, when a filamentous substances which has a fiber diameter falling within the range as specified in the present invention is added to a fermented broth under slow stirring, the filamentous substance comes to be tangled with the filaments of mycelia. Although these filaments of mycelia are highly unstable per se, the closely interwoven structure is stable both physically and chemically. The expression "being physically stable" as used herein means being stable to compression, shear, changes in temperature, etc. On the other hand, the expression "being chemically stable" as used herein means being stable to changes in pH value, changes in solvent composition, etc. It has been also found out that the interwoven structure obtained by filtering the fermented broth composition of the present invention has no compressibility and a high permeability. The formation of the interwoven structure can be confirmed by the use of the phase contrast illumination. When the filamentous substance is a cellulose fiber, it can be clearly confirmed by dyeing the cellulose fiber with a specific dye for cellulose (for example, malachite green) followed by microscopic observation.

The target product can be separated from the fermented broth composition and purified by various techniques and any means or method suitable for the characteristics of the fermented broth composition of the present invention may be selected therefor. Examples of the separation means include filtration, sedimentation and centrifugation while examples of the purification means include washing, recrystallization and liquid chromatography.

The present invention is similar to European Patent No. 402866 and Japanese Patent: Laid-Open No. 49924/1993 in the point of using an organic cellulose material. However these prior arts each relates to a precoat layer which plays an important role in the filtration step and proposes to use a material having specific properties and a specific shape, such as a cellulose material, as a filter-aid suitable for the formation of the precoat layer. Therefore these patents entirely differ in concept from the present invention.

The filamentous substance to be used in the present invention may be an arbitrary one, so long as it is an organic fiber having an average fiber diameter falling within the range as specified in the present invention, being physically harmless and being inert to every substance contained in the fermented broth. From a practical viewpoint, cellulose fibers and chitin fibers may be cited as preferable examples thereof which fit for the purpose of the present invention. Such a cellulose or chitin fiber may be one made from materials of any origin, so long as it has a purity fitting for the purpose of the use. As the filamentous substance to be used in the present invention, a cellulose fiber is particularly preferable and a microfibrilated cellulose is still preferable therefor. Examples of such a cellulose fiber include "Celish KY110A" and "Celish KY110S" (both mfd. by Daicel Chemical Industries, Ltd.)

It is also possible to use a synthetic polymer fiber in the fermented broth composition of the present invention in order to separate and purify the target product. The type of the synthetic polymer fiber is not particularly restricted. Examples thereof include polyolefin fibers, polyacrylic fibers, aromatic polyamides, aromatic polyhydrazides, aromatic polyamide hydrazides, aromatic thiazoles, aromatic oxazoles and aromatic polyesters, and polycarprolactone and polymers of linear aliphatic hydroxy carboxylic acids (for example, polyhydroxybutyric acid and polylactic acid) may be cited as examples thereof.

When the treatment of the cake after the filtration and the application of the fermented broth composition of the present invention in the fields where a high safety is required (drugs, foods, etc.) are taken into consideration, the filamentous substance to be used in the present invention should be a biodegradable one regardless of its being a natural fiber or a synthetic one. In the case of a synthetic fiber, one having a relatively constant fiber diameter can be easily prepared. Although an aramid fiber such as Kevlar^{R} (mfd. by E. I. du Pont de Nemours & Co.) is excellent in filtering characteristics, it is not biodegradable. Such an aramid fiber is not suitable for the present invention from the viewpoint of safety too.

In the present invention, the weight ratio of the actinomyces employed for the fermentation to the above-mentioned biodegradable filamentous organic substance having a fiber diameter falling within a specific range is preferably from 0.01 to 1.0, still preferably from 0.02 to 0.7.

In the present invention, a filamentous substance having an average fiber diameter falling within a specific range is used. Among these substances, those wherein the weight distribution of filaments having an average fiber diameter of from 1.7 to 7.0 µm is at least 50% are particularly preferable and suitably employed in the present invention.

As the filamentous substance, a mixture of various filamentous substances is also usable and the effects of the present invention are not deteriorated thereby, so long as cellulose fibers and chitin fibers having an average fiber diameter within the range as specified above are contained therein in such a total amount as to give a solid content as specified above.

The present invention provides a process for treating a fermented broth composition characterized in that, in any step of such a fermentation process with the use of a microorganism as the one described above, a biodegradable filamentous organic substance having an average fiber diameter of from 0.7 to 10 µm is added to the fermented broth followed by the separation and purification of the fermented broth to thereby obtain the target substance.

The biodegradable filamentous organic substance may be added to the medium before the initiation of the fermentation, in the course of the fermentation, or immediately before the filtration. Then the target substance may be obtained in accordance with a conventional method.

It can be expected that the present invention achieves a filtering performance which is comparable or even superior to the one achieved by the conventional separation and purification method wherein separation is effected through filtration with the use of an inorganic filter-aid (Diatomite, Perlite, etc.) for body feeding and/or precoating. When the target substance is to be extracted from the fermented broth composition according to the present invention, the device and system therefor may be arbitrarily selected without restriction. In the present invention, the biodegradable filamentous organic substance may be added to the fermented broth in any step of the fermentation process.

The waste remaining after filtering the fermented broth composition of the present invention, i.e. the filter cake, is composed exclusively of harmless organic substances. Thus it can be used in, for example, fertilizers and feeds. When it is incinerated, only an extremely small amount of ash is formed. Furthermore, the waste formed in the process of the present invention is applicable to a common biological waste treatment system. Therefore the present invention is advantageous not only in the improvement of the separation and purification efficiencies but also in the recycling of resources, the reduction in the separation and purification costs, the adaption to environmental concerns, etc.

To further illustrate the present invention in greater detail, and not by way of limitation, the following Examples will be given. The properties of the cellulose fibers employed in these Examples are as follows.
- (1) Celish KY110A:: manufactured and distributed by Daicel Chemical Industries, Ltd.,
solid content (cellulose fiber): 35 wt. %,
moisture content: 65 wt. %,
fiber diameter: ranging from 1.0 to 20 µm.
- (1) Celish KY110S:: manufactured and distributed by Daicel Chemical Industries, Ltd.,
solid content (cellulose fiber): 35 wt. %,
moisture content: 65 wt. %,
fiber diameter: ranging from 0.1 to 7 µm.

Table 1 shows the data of the measurement on the fiber diameter distribution of each of the above Celish KY110A and Celish KY110S (counted by using electron microscope photographs).

**[Table 1]**

| Fiber diameter (µm) | | < 0.7 | 0.7-1.7 | 1.7-7 | > 7 |
|---|---|---|---|---|---|
| Celish KY110S | Filament distribution (%) | 95.7% | 3.9% | 0.4% | 0 % |
| | Weight distribution (%) | 16.4% | 7.6% | 76.0% | 0 % |
| Celish KY110A | Filament distribution (%) | 0 % | 0 % | 93.7% | 6.3% |
| | Weight distribution (%) | 0 % | 0 % | 73.7% | 26.3% |
| Powdery cellulose | Filament distribution (%) | 0 % | 0 % | 6.5% | 93.5% |
| | Weigh distribution (%) | 0 % | 0 % | 27.1% | 72.9% |

### Example 1

After conducting the fermentation on an industrial scale (180 hours) by using Penicillium notatum producing pencillin, a sample of the fermented broth was taken out of the fermentor.

The assay level was 36,000 U/ml. Determination after the centrifugation at 3,000 rpm for 10 minutes indicated that the mycelia amounted to 32% by volume and its dry weight was 7.8%.

About 1 ℓ of the sample was mixed with 2 ml of a silicone antifoam, and a large portion of the air was removed in vacuo while slowly stirring. Next, two 300-mℓ portions of the sample were prepared in Erlenmeyer flasks. To one portion (A) were added 3 g of Celish KY110A and 3 g of KY110S. To another portion (B), on the other hand, neither Celish KY110A nor Celish KY110S was added.

Both of these portions were placed on a reciprocal shaker (100 strokes/min) for 5 minutes and then separately filtered in vacuo through two identical Buchner filters each located on a weighed Whatman No. 1 filter paper disk. Then the volume of each filtrate was measured after 5 and 10 minutes and after the filtration.

After weighing the filter paper and the solid matter remaining thereon together (weight 1), the paper and the solid were dried in a drying oven in vacuo and again the filter paper and the solid matter remaining thereon were weighed together (weight 2). The results are as follows.

| (1) | Filtrate | portion A | portion B |
|---|---|---|---|
| | volume after 5 min (ml) | 150 | 135 |
| | volume after 10 min (ml) | 235 | 190 |
| | total volume obtained (ml) | 246 | 230 |
| | penicillin content in filtrate (Mu) | 9.72 | 9.3 |
| | total filtration time (min) | 14 | 29 |
| Note: Toward the end of the filtration, wacks were obtained on the cake of the portion B, which indicates the poor homogeneity of this cake. | | | |

| (2) | Mycelium | portion A | portion B |
|---|---|---|---|
| | weight 1 (before drying; g) | 78.1 | 68.8 |
| | weight 2 (after drying; g) | 26.5 | 20.2 |
| | moisture (1 - 2; g) | 51.6 (66%) | 47.2 (69%) |

Thus it has been proved that when Celish (i.e., a biodegradable filamentous organic substance) is used, filtration can be effected at a substantially elevated rate, a larger amount of the filtrate can be obtained, the total yield of the antibiotic can be elevated, and the moisture content in the cake can be lowered.

### Example 2

3,500 ℓ of a fermented broth, which contained a strain of Claviceps purpurea producing an ergot alkaloid, in a 5,000-ℓ fermentor was subjected to a filtration test.

Four hours before the termination of the fermentation, 105 kg (3% based on the fermented broth) of Celish KY110A was fed into the fermentor. Then the fermentation was continued under the usual aeration and stirring conditions. In the step of harvesting, the fermented broth was collected in a storage tank which was under temperature control under stirring and then acidified in accordance with a usual method. Observation under a microscope proved that cellulose fibers and the hyphae of the mycelia were closely interwoven together.

Filtration was effected on a belt-discharge filter of 5 cm² in filter area under a reduced pressure of 50 to 72 mmHg at a drum rotational speed of 20 revolutions per hour. The filtration was continued for 4 hours while increasing the feed rate of the slurry from 150 to 200 ℓ/m²/hour with the passage of time.

The mycelia recovered in the tank were slurried with 2,000 ℓ of water and then filtered again under the same conditions as those employed above.

The total volume of the combined filtrates amounted to 4,500 ℓ while the total weight of the recovered mycelia amounted to 962 kg. The moisture content was 60%.

These results are comparable to those obtained by using Perlite as the filter-aid or by using Perlite as the precoat layer on a rotary drum filter in vacuo. Thus it has been proved that an excellent filtration performance can be achieved in the present invention without using any filter-aid or effecting precoating.

### Example 3

### (1) Procedure

The device employed in this example was one prepared by modifying a device for measuring the compressibility-permeability of a filtration cake proposed by Grace [see Chem. Eng. Progr., 49, 303 (1953)].

A chromatographic column (height: 40 cm, diameter: 6 cm) having a fritted glass bottom (type F, porosity: 4.5 - 5 µ) was closely fitted to an internal hollow piston having the same fritted glass bottom as the one described above. Then a plate was placed on the top of the internal piston. At the same time, a weight was placed so as to elevate the pressure upon the filter cake.

The test was effected in the following manner. The slurry was poured into the chromatographic column. Then a reduced pressure (50 mmHg) was applied onto the delivery end. When the filtration was completed, the filtrate was collected and the internal piston was inserted into the column. Then the volume of the filtrate and the height of the cake were measured.

Subsequently, the filtrate was poured into the hollow piston and the second filtration was effected. After loading over-weights of 50, 100 and 200 g onto the piston plate, the operation was initiated again. The height of the cake and the volume of the filtrate were measured in each operation.

### (2) Filtration of fermented broth

After conducting the fermentation by using a strain of Streptomyces aureofaciens producing tetracycline, oxalic acid was added to thereby precipitate calcium ions and the fermented broth was sampled in accordance with the procedure. The dry weight of the solid matter after the filtration was 6.0%. The filtration was effected by using the device described in the above (1). Table 2 shows the results thus obtained.

Two 1-ℓ portions (A and B) were prepared from the fermented broth sample. To the portion A was added 30 g of Celish KY110A and thus the dry weight was increased to 9.0%. On the other hand, no Celish KY110A was added to the portion B.

**[Table 2]**

| | | | Portion A | | Portion B | |
|---|---|---|---|---|---|---|
| | | | Cake ht. (cm) | Filtrate vol. (ml) | Cake ht. (cm) | Filtrate vol. (ml) |
| 1st filtrn. | | | 48.5 cm | 750 ml (clear) | 13 cm | 625 ml (clear) |
| 2nd filtrn. | Immediately after | | 9.5 cm | 730 ml (clear) | 15 cm | 570 ml (clear) |
| | Loaded | 50 g | 8.0 cm | 755 ml (clear) | 11 cm | 685 ml (clear) |
| | | 100 g | 7.0 cm | 795 ml (clear) | 10 cm | 685 ml slowly filtered, incompletely turbid |
| | | 200 g | 6.5 cm | 810 ml (highly turbid) | hardly filtrable | |

By adding Celish, a smaller compressibility of the cake and a high permeability capable of giving an improved filtration performance can be achieved and a filtrate of better qualities can be obtained even under a severe mechanical stress.

### Example 4

A sample of the fermented broth of Streptomyces erythraeus producing erythromycin showed an assay level of 7,000 mg/ml and a dry weight of 5% after the fermentation. In a microscopic examination, somewhat enlarged mycelia and hyphae with limited branching were observed.

Two 300-ml portions were prepared from the sample. To one portion (A) was added Celish KY110A while one was added to another portion (B) followed by the comparative filtration in the same manner as the one described in the above Example 1. The results are as follows:

| filtrate | Portion A | Portion B |
|---|---|---|
| volume after 5 min (ml) | 120 | 115 |
| volume after 10 min (ml) | 205 | 180 |
| total volume obtained (ml) | 253 | 225 (incomplete) |
| total filtration time (min) | 17 | 34 |

### Example 5

A fermented broth was harvested from a fermentor on an industrial scale for producing cephalosporin C by using a variant of Cephalosporium achremonium.

Two 300-ml portions were prepared from the sample. To one portion (A) were added Celish KY110A and Celish KY110S (1 : 1, the total content: 3%) while none was added to another portion (B) followed by retention.

These portions were subjected to comparative filtration with the use of Buchner filters in the same manner as the one described in the above Example 1. The results are as follows:

| filtrate | Portion A | Portion B |
|---|---|---|
| volume after 5 min (ml) | 170 | 140 |
| volume after 10 min (ml) | 220 | 195 |
| total volume obtained (ml) | 240 | 225 |
| total filtration time (min) | 12 | 20 |

### Example 6

One metric ton of mycelis (A) obtained by precoating a rotary drum filter of a fermentor tank, where fermentation with a species of Claviceps producing Ergokryptine had been conducted, with Perlite, adding 2% of Perlite to the slurry and then filtering the same were incinerated at 1,100°C.

One metric ton of mycelia (B) obtained from a rotary belt-discharge filter of the same fermentor tank as the above one after adding 2% of Celish KY110S and then filtering were incinerated by the same method.

The incinerator employed herein was a batch-fired incinerator for laboratory use having a grate area of 4 m². Before the operation, the mycelia A contained 60% of moisture while the mycelia B contained 65% of moisture.

The incineration was effected at a feeding rate of 80 kg/hr/m² (grate area) while supplying air in an amount sufficient for maintaining the carbon monoxide concentration in the exhaust at the minimum level.

The ash remaining in the incinerator was collected in an ash chamber. Thus 128 kg of ash was collected in the case of the mycelia A while 1.95 kg of ash was collected in the case of the mycelia B.

### Example 7

A fermented broth containing 20% by weight of solid matters was obtained from the fermentation medium, wherein fermentation by Streptomyces erythraeus had been effected for 180 hours, and cooled to 10°C. Then 3% (dry weight/volume) of each filamentous substance as listed in Table 3 was added to the fermented broth and homogenized by stirring for 5 minutes. Next, the sample was filtered through a Buchner filter under the conditions as specified in Table 3 and the filtration performance was evaluated. Table 3 shows the results.

**[Table 3]**

| Filamentous substance | | Av. fiber length (µm) | Av. fiber diameter (µm) | Biodegradability | Filtrn. rate (ℓ/m²/hr) | State of filtrate | Solid matter in filter cake (%; wt./vol) |
|---|---|---|---|---|---|---|---|
| Invention | Celish KY110A | 500 | 3.0 | yes | 400 | slightly turbid but nonproblematic in practice | 40 |
| | Celish KY110S | 500 | 0.8 | yes | 300 | clear | 40 |
| Comparison | chitin | 350 | 0.1 | yes | 250 | clear | 35 |
| | cellulose powder | 160 | 20 | yes | 150 | clear | 25 |
| | polyacrylamide | 750 | 0.1 | no | 200 | clear | 30 |
| | Kevlar*¹ | 500 | 20 | no | 500 | turbid | 45 |

| | | | | | | | |
|---|---|---|---|---|---|---|---|
| *1: registered trademark, E. I. du Pont de Nemours & Co. | | | | | | | |

## Claims

1. A fermentation broth composition comprising a biodegradable, filamentous organic substance having an average fiber diameter of from 0.7 to 10 µm and a filamentous actinomyces as a microorganism to employ for the fermentation.

2. The composition as claimed in Claim 1, wherein said actinomyces is Streptomyces erythraeus or Streptomyces aureofaciens.

3. The composition as claimed in Claim 1, wherein a weight ratio of the actinomyces to the biodegradable filamentous organic substance ranges from 0.01 to 1.0.

4. The composition as claimed in Claim 1, wherein said biodegradable filamentous organic substance is a cellulose fiber or a chitin fiber.

5. The composition as claimed in Claim 1, wherein a weight distribution of filaments having an average fiber diameter of from 1.7 to 7.0 µm in said biodegradable filamentous organic substance is at least 50%.

6. A process for conducting a fermentation reaction with a filamentous actinomyces as the microorganism, characterized by adding a biodegradable filamentous organic substance having an average fiber diameter of from 0.7 to 10 µm to the microorganism, then conducting the fermentation and isolating desired components from the product mixture by way of filtration.
